# EUROPÄISCHE PATENTSCHRIFT

(11) **EP 0 522 125 B1**
(45) Veröffentlichungstag und Bekanntmachung des Hinweises auf die Patenterteilung: **08.11.1995**
(21) Anmeldenummer: 92903228.2
(22) Anmeldetag: 24.01.1992
(51) Int. Cl.: A61B 17/32

(54) **ZERVIX-STANZE**
CERVICAL BIOPSY PUNCH
EMPORTE-PIECE POUR BIOPSIE DU COL UTERIN

(30) Priorität: 25.01.1991 DE 9100873 U; 02.10.1991 DE 9112303 U; 19.11.1991 DE 9114443 U
(43) Veröffentlichungstag der Anmeldung: 13.01.1993
(73) Patentinhaber: WISAP GESELLSCHAFT FÜR WISSENSCHAFTLICHEN APPARATEBAU MBH, D-82054 Sauerlach (DE)
(72) Erfinder: SEMM, Kurt, D-2300 Kiel (DE)
(74) Vertreter: Heim, Hans-Karl, Dipl.-Ing.
(86) Internationale Anmeldenummer: EP9200150
(87) Internationale Veröffentlichungsnummer: WO9212676

(56) Entgegenhaltungen:
- WO-A-88/10098
- DE-A- 1 905 232
- DE-A- 2 622 850
- DE-U- 9 100 873
- FR-A- 853 349
- GB-A- 2 099 703
- US-A- 4 243 048

## Beschreibung

Die Erfindung betrifft eine Zervix-Stanze zur Aushülsung der Zervix-Fascia von uterinem Gewebe für die Gebärmutterexstirpation.

Bisher ist es üblich, die Gebärmutter bei der Indikation gutartiger Veränderung total abzusetzen.

Im Rahmen des Versuchs, die Gebärmutter auf endoskopischem Wege zu entfernen, trifft man jedoch auf Schwierigkeiten der Aushülsung der Zervix aus dem perizervikalen sehr gefäßreichen Gewebe.

Aus der WO-A-88/10 098 ist ein Instrument zur intrazervikalen Hysterektomie mit einer Führungseinrichtung bekannt, die aus einem Mittel zur Verankerung besteht, welches dem Schneidbereich gegenübersteht und einen zylindrischen Einschnitt bzw. eine Schneide aufweist, welche mit dem Schneidbereich des Grundkörpers zusammenwirkt. Die bekannte Zervix-Stanze ist als proximalseitig geschlossene, rohrartige Buchse ausgebildet, die proximalseitig eine starke Durchmesserreduzierung aufweist, durch die ein mit Gewinde versehener Führungsstab hindurchreicht. Ein distalseitiger Schneidbereich ist als keilförmige Abschrägung ausgebildet.

Außerdem sind aus der DE-A 26 22 850 und aus der DE-A 1 905 232 Probeentnahmegeräte bekannt, die einen gezahnten und einen mit einem Wellenschliff versehenen Schneidbereich aufweisen. Diese Probeentnahmegeräte sind für einen endochirurgischen Eingriff in keiner Weise geeignet.

Der Erfindung liegt die **Aufgabe** zugrunde, ein medizinisches Instrument zu schaffen, welches bei einer besonders einfachen konstruktiven Gestaltung die Entfernung uterinen Gewebes in einem genau definierten Bereich der Zervix und auf besonders schonende Weise gewährleistet.

Diese Aufgabe wird mit der Zervix-Stanze gemäß Anspruch 1 gelöst. Vorteilhafte Weiterbildungen der Erfindung sind Gegenstand der Unteransprüche.

Erfindungsgemäß ist bei einer Zervix-Stanze mit einem hohlzylindrischen Rundkörper, welcher am distalen Ende eine Öffnung mit einem Schneidbereich und am proximalen Ende einen Eingriffsbereich zur Rotation des Grundkörpers relativ zu einer Führungseinrichtung aufweist, wobei der Grundkörper wenigstens in einem proximalen Teil an die Führungseinrichtung angepaßt ist, als Grundkörper ein Rundrohr vorgesehen, welches einen über die gesamte Länge gleichbleibenden Außendurchmesser aufweist und mit einem Schneidbereich an der distalen Öffnung versehen ist, der als umlaufender Wellenschliff ausgebildet ist.

Die Gefahr einer malignen Entartung des nach suprazervikaler Gebärmutterentfernung verbliebenen Genitalschlauches geht fast ausschließlich von der Umwandlungszone im Bereich der Portio cervikalis, dem Endothel der Zervix und dem Endometrium aus. Nachdem bislang die Fascia cervicalis von aussen aus dem umgebenden Gewebe, d.h. der Arteria uterina, den Venae uterinae, dem Ureter, dem periurethalen Gewebe und dem entsprechenden Plexus nervosus ausgehülst wurde, ist es nun durch die vorliegende Erfindung möglich, die Zervix-Fascie vom uterinen Gewebe innerhalb der Zervix auszuhülsen.

Hierfür ist der Durchmesser der Zervix vorab durch transvaginalen Ultraschall sehr genau (auf den Millimeter) zu bestimmen. Sodann wird ein Führungsstab in den Uterus eingeführt, der den Uterus längs ausrichtet und den Uterusfundus möglichst zentral durchstößt. Danach wird der Grundkörper der Zervix-Stanze auf den Führungsstab aufgeschoben und unter einer Drehbewegung in den Zervix-Kanal eingeführt, um diesen in entsprechendem, vorgegebenen Abstand zur Fascia cervicalis zylinderförmig auszustanzen. Der verbleibende Hohlzylinder, der später als Drainage wirkt, läßt sich nach transabdominaler oder pelviskopischer Ligatur in Bezug auf spätere maligne Degeneration risikolos belassen. Der Patientin bleiben somit zum einen sämtliche vaginalen Funktionen erhalten und zum anderen besteht keine transvaginale abdominale Infektionsgefahr. Nach dem Herausziehen des Grundkörpers zusammen mit dem ausgestanzten Gewebe können offene Gefäße durch Einführen einer zylinderförmigen Koagulationssonde, eines Hämostasers, verschlossen werden.

Der dünnwandige hohlzylindrische distale Teil des Grundkörpers hat eine distale Öffnung, die mit einer Schneide versehen ist. Die Schneide weist einen sehr scharfen Wellenschliff auf, der bei einer Drehbewegung des Grundkörpers eine exakte Schnittführung im uterinen Gewebe bewirkt, ohne daß das verbleibende Gewebe zu stark traumatisiert wird. So läßt sich uterines Gewebe aushülsen, ohne daß die Zervix-Fascie bei dem Vorgang deformiert wird. Am distalen Ende der Zervix-Stanze können auf der Innenseite des hohlzylindrischen Grundkörpers scharfkantige Klappelemente ausgebildet sein, die bei einem Zurückziehen des Grundkörpers in proximaler Richtung von ihrer axialen Lage in eine radiale Lage ausklappen und dabei eine Abtrennung des ausgestanzten uterinen Gewebes am distalen Ende des ausgestanzten Gewebepfropfens bewirken.

Der Grundkörper weist an seinem proximalen Ende eine sich radial erstreckende kreisrunde Griffplatte auf, deren Rand profiliert gestaltet ist, um eine leichtere Bedienung vor allem hinsichtlich der Einstellung einer Drehbewegung beim Schneidvorgang zu ermöglichen.

Die distale Hälfte der Länge des Grundkörpers ist vorzugsweise als dünnwandiger Hohlzylinder zur Aufnahme des auszuhülsenden uterinen Gewebes ausgebildet, während die proximale Hälfte der Länge des Grundkörpers eine Durchbohrung mit einem Innendurchmesser aufweist, der nur wenig über dem Außendurchmesser des Führungsstabes liegt, also paßgenau ausgelegt ist, so daß der Führungsstab in diesem proximalen Bereich lediglich für eine axiale Bewegung geführt ist.

Der Übergang des Innendurchmessers vom proximalen zum distalen Teil ist dabei möglichst fließend, möglichst konisch ausgebildet, um ein Anheften von entferntem Gewebe an Ecken oder Kanten zu vermeiden.

Es ist jedoch auch möglich, den gesamten Grundkörper dünnwandig zu gestalten und im proximalen Teil Zentrierringe anzuordnen, die eine zentrale Ausrichtung des Führungsstabes in dem Grundkörper sicherstellen sollen.

Der Führungsstab weist vorzugsweise eine axiale Längsnut auf, die in einem bestimmten Abstand, vorzugsweise im Bereich von 6 bis 60 mm vor der Spitze endet, damit keine Luft aus dem Pneumoperitoneum beim Durchstechen des Uterusfundus entweicht. Die Längsnut soll ein Entweichen von Luft aus dem zervikalen Bereich sicherstellen und damit einer Behinderung des Aushülsungsvorgangs oder einer Traumatisierung der Zervix-Fascie bei der Aushülsung entgegenwirken.

Die gesamte Zervix-Stanze mit Führungsstab ist aus V2A-Stahl gefertigt und somit sowohl thermisch als auch chemisch sterilisierbar.

Der Grundkörper der Zervix-Stanze kann in verschiedenen Durchmessern, z.B. 10 mm bis 30 mm, hergestellt werden. Sie hat eine Länge von vorzugsweise 30 cm, während der Führungsstab deutlich länger als der Grundkörper, z.B. 60 cm, ausgebildet sein kann. Der Führungsstab kann nach der Aushülsung von Gewebe zum zentrierten Aufschieben einer ebenfalls hohlzylindrisch ausgebildeten Koagulationssonde verwendet werden, um eine Blutstillung des verbliebenen uterinen Gewebes innerhalb der Zervix-Fascie zu ermöglichen.

Der am vordersten Rand seiner Distalöffnung mit einem Wellenschliff versehene Grundkörper, der auch als Morcellator bezeichnet werden kann,, ist vorteilhafterweise mit einem kontinuierlichen Wellenschliff ausgestattet. In dieser Konfiguration zeigt der Abdruck dieses Wellenschliffs in einer Ebene mehrere, über den Umfang gleichmäßig verteilte Kreisbogenabschnitte, die voneinander sehr klar beabstandet sind. Beispielsweise würden bei vier Wellenvorsprüngen und vier dazwischen liegenden, leicht axial in der Umfangsfläche zurückgeführten Wellentälern Bogensegmente etwa im Bereich von 40 bis 45^{o} entstehen. Die Schärfungszone des Wellenschliffs kann über-den gesamten Umfang der-Distalöffnung vorhanden sein. Zweckmäßigerweise kann diese Schärfungszone jedoch im Übergangsbereich zwischen dem Wellenvorsprung und dem Wellental besonders scharf gestaltet sein, so daß zunächst eine axiale Fixierung des Gewebes und erst anschließend durch die Rotationsbewegung des Grundkörpers das Morcellieren von Gewebezylindern bzw. Gewebehülsen durchgeführt wird.

Der Wellenschliff kann gegebenenfalls jedoch auch diskontinuierlich in Art einer schuppenförmigen Anordnung um den Mantelumfang an der Distalöffnung vorgesehen sein.

Obwohl der Grundkörper zusammen mit dem proximalseitig vorgesehenen Rundgriff bzw. Rundgriffring einstückig oder unlösbar miteinander ausgelegt ist, erreicht man mit einem austauschbaren Grundkörper im Rundgriffring weitere Verbesserungen. So ermöglicht diese Austauschbarkeit, ein Instrumentenset der Zervix-Stanze z.B. mit mehreren Grundkörper- bzw. Morcellator-Rohren auszustatten. Im Falle der Abnutzung des insbesondere am Außenmantel vorgesehenen Wellenschliffs des Schneidbereichs des Morcellators kann rasch und problemlos ein neues Morcellator-Rohr eingesetzt werden. Auch sind Abstufungen im Innen- und Außendurchmesser des Grundkörpers bzw. Morcellator-Rohres hierdurch möglich.

Die Fixierung des austauschbaren Morcellator-Rohres kann in einfacher Weise durch einen in den Innenbereich des Rundgriffringes einsetzbaren Klemmring realisiert werden. Ein derartiger Klemmring kann einseitig geschlitzt sein und aus Materialspannungsgründen auf der gegenüberliegenden Seite eine etwa achsparallel verlaufende Nut im Außenumfang aufweisen, so daß dieser Klemmring über diametral angreifende Inbus- bzw. Maulschrauben den mit Paßsitz eingeschobenen proximalen Bereich des Grundkörpers festklemmt. In proximaler Richtung liegen dabei sowohl der Klemmring als auch das Grundkörper-Rohr gegen einen den Innendurchmesser des Grundkörper-Rohres aufweisenden Ringkragen an.

Als Alternative zur Handbetätigung für die Rotationsbewegung des Grundkörpers beim Ausstanzen oder Aushülsen von Gewebe besteht auch die Möglichkeit, über einen kleinen Motor, der elektrisch, pneumatisch oder hydraulisch angetrieben ist, die Rotationsbewegung auf den Grundkörper aufzubringen. Hierzu kann die Antriebswelle des Motors über einen drehfest zum Außenumfang des Grundkörper-Rohres angebrachten Zahnring mit diesem in Eingriff stehen und beispielsweise über beidseitig vom Zahnring vorgesehene kleine Lagerringe die Fixierung des Motors am Grundkörper-Rohr durchgeführt sein.

Die Erfindung wird nachfolgend beispielsweise anhand der schematischen Zeichnung beschrieben. In dieser zeigen:
- Fig. 1: einen Längsschnitt durch einen rotationssymmetrischen Grundkörper einer Zervix-Stanze mit einer distalen Schneide;
- Fig. 2: einen Längsschnitt durch einen Führungsstab;
- Fig. 3: einen vergrößerten Teil eines Längsschnittes eines Grundkörpers in einer weiteren Ausführungsform;
- Fig. 4: einen Längsschnitt durch den proximalen Teil eines Grundkörpers mit einem Schraubeinsatz;
- Fig. 5: einen Längsschnitt durch eine Zervix-Stanze mit Grundkörper und Führungszylinder;
- Fig. 6: eine schematische Darstellung eines weiteren Ausführungsbeispiels der Zervix-Stanze mit innerem Führungszylinder, wobei die Rotationsbewegung des Grundkörpers mittels eines Motors aufgebracht wird, und
- Fig. 7: eine schematische Draufsicht auf einen proximalseitig am Grundkörper aufbringbaren Rundgriffring mit innerem Klemmring.

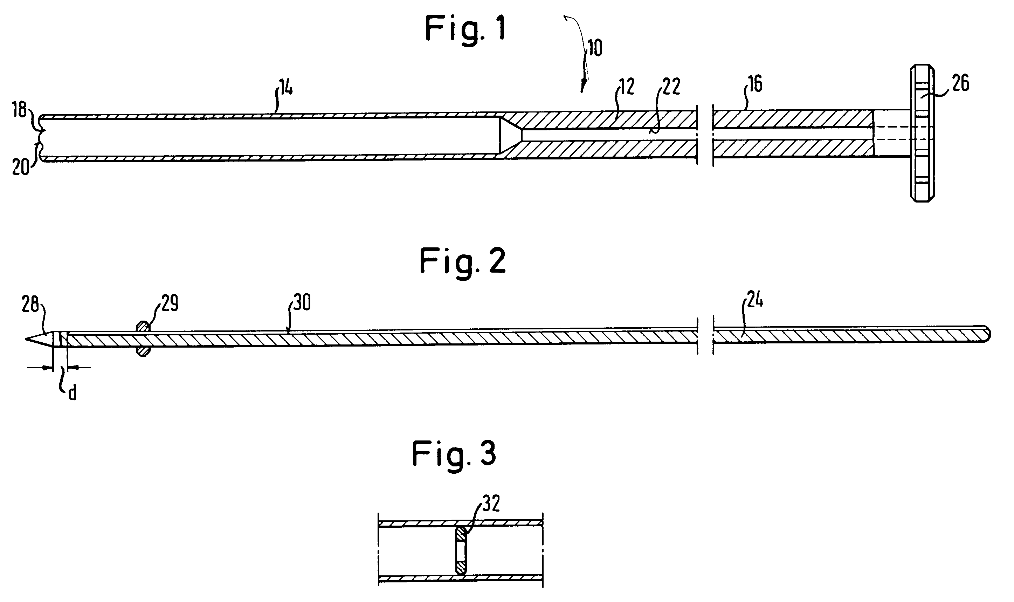

In Fig. 1 ist eine Zervix-Stanze 10 mit einem hohlzylindrischen rotationssymmetrischen Grundkörper 12 dargestellt.

Der Grundkörper 12 ist über seine Länge in zwei Bereiche 14, 16 unterteilt, die jeweils eine unterschiedliche Wandstärke aufweisen. Die distale Hälfte 14 des Grundkörpers 12 ist als dünnwandiger Hohlzylinder ausgebildet, dessen Distalöffnung 18 einen Schneidbereich 20 aufweist, der mit einem umlaufenden Wellenschliff in Art eines Schneidkranzes oder einer Schneidkrone versehen ist, die z.B. etwa zwölf Schneidwellen haben kann. Der proximale Teil 16 des hohlzylindrischen Grundkörpers 12 hat eine zentrische Durchbohrung 22 mit einem im Vergleich zur distalen Hälfte 14 sehr viel geringeren Durchmesser. Der Innendurchmesser der Durchbohrung 22 entspricht im wesentlichen dem Außendurchmesser des in Fig. 2 dargestellten Führungsstabes zuzüglich eines geringen Spiels, um eine klemmfreie axiale Verschiebung des Führungsstabs 24 in der Durchbohrung 22 zu gewährleisten. Das proximale Ende des proximalen Teils 16 des Grundkörpers 12 trägt einen Griffbereich 26, der als radial verlaufende kreisrunde Griffplatte ausgebildet ist. Die Griffplatte ist drehfest mit dem Grundkörper 12 verbunden und weist einen profilierten Rand, z.B. eine Riffelung auf, um den Grundkörper 12 durch die Bedienung der Griffplatte 26 in eine Drehbewegung versetzen zu können, auch wenn die Handhabung erschwert und damit die Griffigkeit herabgesetzt ist.

Das distale Ende des Führungsstabes 24 ist als kegelförmige Spitze 28 ausgebildet. In einem Abstand d von z.B. 6 bis 60 mm von dieser Spitze 28 ist eine axiale Längsnut 30 in dem Führungsstab 24 ausgebildet. Diese Nut 30 soll einen Gasaustausch zwischen Zervix und der Umgebungsluft ermöglichen, um einem Überdruck beim Einführen der Zervix-Stanze in den Uterus entgegenzuwirken.

Auf den Führungsstab 24 kann ein Führungsring 29 aufgebracht sein, der zu seiner äußeren Umfangsfläche hin konifiziert ist. Der Außendurchmesser dieses Führungsringes 29 ist dem Innendurchmesser des hohlzylindrischen Grundkörpers 12 der Zervix-Stanze 10 angepaßt und dient zur Zentrierung des Grundkörpers, der in seiner Ausbildungsform gemäß Fig. 1 nur in seinem proximalen Bereich 16 an dem Führungsstab 24 geführt ist. Durch diesen Führungsring 29 wird demnach eine koaxiale Ausrichtung des Grundkörpers 12 bezüglich des Führungsstabes 24 erreicht.

Es ist auch möglich, nach dem Einführen des Führungsstabes 24 in den Uterus und vor dem Aufschieben der Zervix-Stanze 14 einen Führungsring in Art einer Scheibe auf den Muttermund haftend aufzubringen. Darüber wird dann der Schneidkranz der Zervix-Stanze präzise geführt, da sonst gerade in der Erstphase des Ausstanzens des zylindrischen Gewebes am Uterus eine Abweichung zwischen Führungsstab und Zervix-Stanze gerade im vorderen Hohlbereich denkbar wäre.

Fig. 3 zeigt einen relativ zu den Fig. 1 und 2 vergrößerten Teilausschnitt eines Längsschnittes durch eine Zervix-Stanze entsprechend Fig. 1 oder in einer anderen Ausführungsform.

Während die Führung des Führungsstabes 24 in der Zervix-Stanze 10 in erster Linie durch die an den Außendurchmesser des Führungsstabes 24 angepaßte Innenbohrung 22 des Grundkörpers 12 gewährleistet wird, ist eine Führung des Führungsstabes 24 in dem Grundkörper einer Zervix-Stanze auch durch einen oder mehrere Führungsringe 32 möglich, die entweder axial verschieblich oder fest positioniert zwischen dem Grundkörper und dem Führungsstab angeordnet werden.

Bei der Zervix-Stanze 10 aus Fig. 1 ist eine Verwendung von Zentrierringen 32 im distalen Teil 14 des Grundkörpers 12 möglich. Dieser Zentrierring 32 schiebt sich dann beim Ausstanzen von uterinem Gewebe in Richtung des proximalen En-Endes des distalen Teils 14.

Der Übergang zwischen den unterschiedlichen Innendurchmessern des distalen Teils 14 und des proximalen Teils 16 des Grundkörpers 12 ist vorzugsweise fließend, z.B. konisch ausgebildet, um eine Reinigung des Instruments und ein Festhaften von Gewebe an Ecken und Kanten zu vermeiden.

Bei einem Eingriff zur Entfernung von uterinem Gewebe wird zuerst der Führungsstab 24 in den Uterus eingeführt, wobei der Uterus z.B. durch weitere endoskopische Geräte koaxial zum Führungsstab 24, d.h. gestreckt, ausgerichtet wird. Mit der Spitze 28 des Führungsstabs 24 wird dann der Uterusfundus im Zentrum des Uterus durchstoßen. Hierbei wird darauf geachtet, daß die Spitze 28 den Uterusfundus nicht weiter als die Länge d durchstößt, damit über die Nut 30 im Führungsstab 24 kein Gas aus dem Pneumoperitoneum des Abdominalbereichs in die Umgebung entweicht. Da der Führungsstab 24 in etwa eine Länge von 60 cm aufweist, ragt er weit aus dem Vaginalbereich heraus. Auf diesen herausragenden Teil des Führungsstabes 24 wird der Grundkörper 12 der Zervix-Stanze 10 mit dem distalen Ende voran aufgeschoben. Der Führungsstab 24 gelangt dabei in die innere Führungsbohrung 22 des proximalen Teils 16 des Grundkörpers 12, wodurch dieser an dem Führungsstab 12 zentriert wird.

Mittels Ultraschall, Röntgen oder optischer Verfahren ist vorher der Durchmesser der Zervix festzustellen, wodurch der Außendurchmesser des Grundkörpers 12 genau festgelegt werden kann. Der Grundkörper 12 wird nun unter vorzugsweise permanenter Drehbewegung in den Uterus ausstanzend hineingedreht oder -geschoben, wodurch vor der Distalöffnung 18 befindliches uterines Gewebe mittels der Schneide 20 abgetrennt bzw. ausgestanzt wird. Das abgetrennte Gewebe gelangt dann in den Bereich zwischen dem distalen Teil 14 des Grundkörpers 12 und dem Führungsstab 24.

Wenn die Distalöffnung 18 den Uterusfundus erreicht hat, wird die Zervix-Stanze mittels des Griffteils zurückgezogen, wobei darauf zu achten ist, daß das abgetrennte Gewebe, das sich in dem distalen Teil 14 des Grundkörpers 12 befindet, mit aus dem Uterus herausgezogen wird. Hierfür können hinter der Distalöffnung 18 Klappen mit scharfen Kanten angeordnet sein, die von einer axialen Lage in eine radiale Lage umklappbar sind. Beim Zurückziehen der Zervix-Stanze 12 werden diese klappbaren Teile in eine radiale Lage ausgeklappt und trennen somit das im distalen Teil 14 befindliche uterine Gewebe im Bereich der Distalöffnung 18 von dem verbleibenden Uterusgewebe ab.

Nach dem Herausziehen der Zervix-Stanze kann eine hohlzylindrische Koagulationssonde, ein Hämostaser, über den verbleibenden Führungsstab 24 geschoben werden. Mit diesem Hämostaser können offene Gefäße des verbleibenden uterinen Gewebes mittels Thermobehandlung geschlossen und eine Blutstillung herbeigeführt werden.

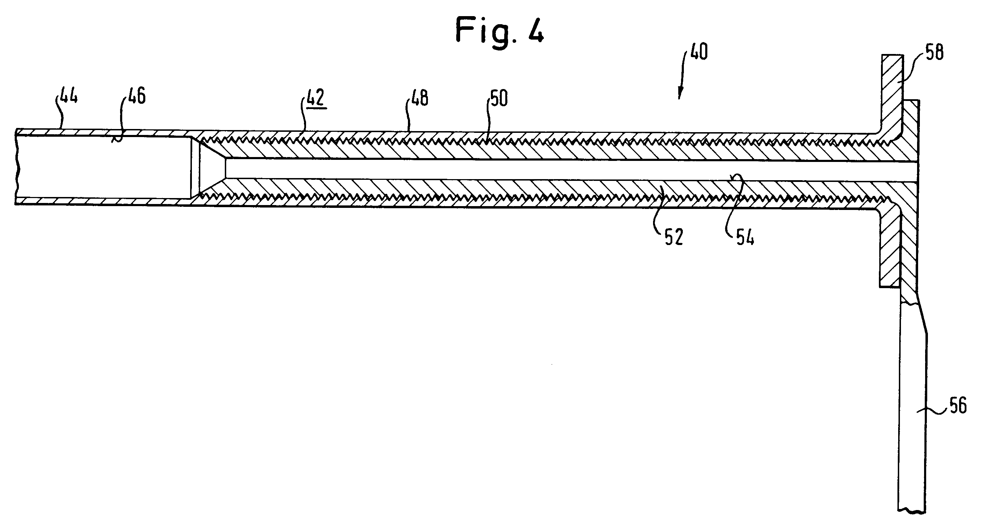

Fig. 4 zeigt eine Zervix-Stanze 40 mit einem Grundkörper 42, der über seine gesamte Länge als dünnwandiger Hohlzylinder ausgebildet ist. In seinem distalen Teil 44 ist die Innenwand 46 des Grundkörpers 42 für die Aufnahme von auszustanzendem Gewebe glatt ausgebildet. Der proximale Teil, insbesondere die proximale Hälfte 48 des Grundkörpers 42, weist an der Innenwand des Hohlzylinders ein Schraubgewinde 50 auf, das in ein komplementäres Außengewinde am Außenumfang eines als Führungszylinder ausgebildeten Schraubeinsatzes 52 eingreift, der wiederum eine Bohrung 54 für die Führungsstange 24 aufweist. Der Schraubeinsatz 52 ragt an seinem proximalen Ende aus dem proximalen Teil 48 des Grundkörpers 42 heraus. An dieser Stelle weist der Schraubeinsatz 52 ein sich radial erstreckendes Griffteil 56 auf, das dazu dient, den Schraubeinsatz 52 rotationsfest zu halten, während der Grundkörper 42 an seiner proximalen Griffplatte 58 in den Uterus hineingeschraubt wird. Der Schraubeinsatz 52 bleibt dabei in axialer Richtung fest mit dem Führungsstab 24 verbunden, der wiederum im Uterusfundus fixiert ist. Durch das Festhalten des Schraubeinsatzes 52 und die Drehbewegung des Grundkörpers 42 relativ zum Schraubeinsatz 52 wird somit einer Drehbewegung, die der Schneide am distalen Ende der Zervix-Stanze 40 zu einer entsprechenden Schneidwirkung verhilft, eine definierte axiale Bewegung überlagert.

Es ist selbstverständlich möglich, die Länge des Schraubeinsatzes 52 so zu wählen, daß dieser sich über die gesamte Länge des Grundkörpers 42 erstreckt, bzw. distal sogar etwas aus dem Grundkörper 42 hervorsteht. Das Innengewinde 50 könnte hierbei über die gesamte Länge in dem Grundkörper 42 angeordnet sein. Es wird jedoch vorzugsweise ein distaler Bereich des Grundkörpers gewindefrei gehalten. Wenn der Schraubeinsatz 52 und der Grundkörper 42 ein komplementäres Gewinde nur im proximalen Teil aufweisen, müßte der Außendurchmesser des Schraubeinsatzes 52, der als Führungszylinder dient, auf den Grund des Schraubgewindes verringert sein, damit sich der mit dem Innengewinde versehene Teil des Grundkörpers über den gewindelosen Teil des Schraubeinsatzes schieben kann. Vorzugsweise haben der Grundkörper und der Schraubeinsatz bzw. der Führungszylinder eine Länge von 250 mm, der Außendurchmesser des Grundkörpers beträgt vorzugsweise 10, 15 oder 20 mm.

Die Steigung des Gewindes 50 kann etwa 8 mm betragen. Durch die Umdrehungsgeschwindigkeit des hohlzylindrischen Grundkörpers 42 um den Führungszylinder kann somit die Vortriebsgeschwindigkeit des Grundkörpers und damit die Einschnittgeschwindigkeit des distalen Schneidkranzes 20 am Grundkörper gewindegesteuert durchgeführt werden und auch von Assistenzpersonal relativ exakt eingehalten werden.

Das radiale Griffteil 56 kann weiterhin als Fixierung für eine nicht dargestellte Kugelzange ausgebildet sein, mit der der Vaginalbereich während des Eingriffs aufgespannt werden kann.

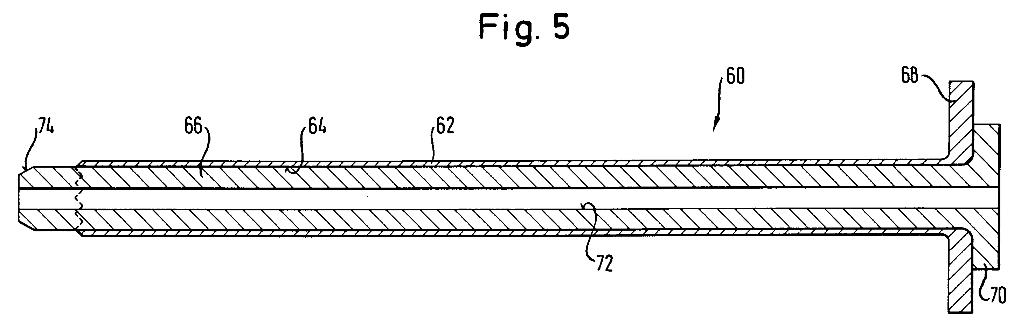

Fig. 5 zeigt eine Zervix-Stanze 60, bestehend aus einem dünnwandigen hohlzylindrischen Grundkörper 62, dessen Innenwand 64 zur Aufnahme eines hohlzylindrischen Führungszylinders 66 glatt ausgebildet ist. Der Grundkörper 62 hat an seinem proximalen Ende eine radial verlaufende Griffplatte 68 zur Bedienung des Instruments. Das proximale Ende des Führungszylinders 66 ist ebenfalls radial in Art einer Griffplatte 70 aufgeweitet. Im völlig eingeschobenen Zustand liegt der Führungszylinder 66 mit seiner Griffplatte 70 an der Griffplatte 68 des Grundkörpers 62 an.

Der Führungszylinder 66 weist eine axiale Durchbohrung 72 als Führung für die Aufnahme eines Führungsstabes 24 auf.

In völlig eingeschobenem Zustand gemäß Fig. 5 ragt der Führungszylinder 66 an seinem distalen Ende über den Grundkörper 62 hinaus und ermöglicht hierdurch eine Zentrierung des Grundkörpers im Bereich des Uterusmundes, insbesondere bei Beginn des operativen Eingriffes.

Die am distalen Ende des Führungszylinders 66 befindlichen Kanten zwischen Stirnfläche und Mantelfläche sind vorzugsweise für eine bessere Führung der Zervix-Stanze 60 koniziert ausgebildet.

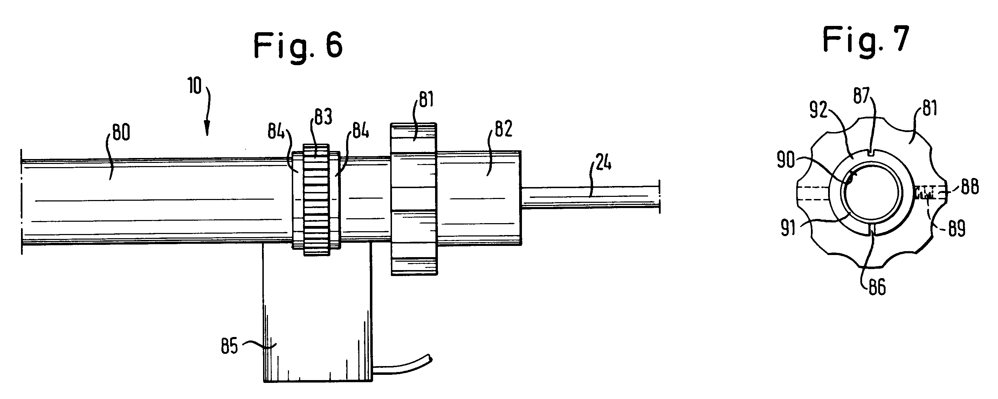

In Fig. 6 ist bruchstückartig der proximale Bereich einer Zervix-Stanze 10 mit Motorantrieb dargestellt. Der Grundkörper bzw. das Morcellator-Rohr 80 ist proximal mit einem Rundgriffring 81 ausgestattet. Dieser Rundgriffring 81 liegt in der Darstellung an einem Rundgriffring 82, der z.B. starr mit dem im Morcellator-Rohr 80 vorgesehenen Führungszylinder verbunden ist. Beide Rundgriffringe 81, 82 sind mit Randvertiefungen ausgestattet, um eine bessere manuelle Betätigung zu ermöglichen. Durch den Führungszylinder mit Rundgriffring 82 hindurch reicht der proximal herausragende Führungsstab 24.

Zur Verbesserung des Komforts bei der Betätigung der Zervix-Stanze und einen präzisen, sicheren Schneid- bzw. Ausstanzvorgang ist im Beispiel nach Fig. 6 ein Motorantrieb über den Motor 85 dargestellt. Dieser elektrisch betätigte Motor 85 greift z.B. über ein Schneckengewinde in einen drehfest auf dem Außenmantel des Morcellator-Rohres 80 aufgebrachten Zahnkranz 83 ein. Beidseitig von diesem Zahnkranz sind im Beispiel Kugellagerringe 84 aufgebracht, an denen der Motor 85 befestigt ist.

Auf diese Weise ist es möglich, z.B. mit einer Rotationsbewegung von 60 bis 80 Umdrehungen pro Minute rasch und äußerst genau einen Aushülsvorgang der Zervix durchzuführen.

In Fig. 7 ist in axialer Draufsicht in Richtung zum proximalen Ende der Rundgriffring 81 ohne Morcellator-Rohr 80 dargestellt. In der Innenöffnung des Rundgriffringes 81 ist relativ paßgenau ein Klemmring 92 plaziert. Dieser Klemmring 92 schließt flächenbündig mit dem Rundgriffring 81 ab und liegt proximalseitig gegen einen Ringkragen 91, gegen den auch das proximale Ende des Morcellator-Rohres 80 anschlägt, an. Der Klemmring 92 ist geschlitzt (bei 86) und weist diametral gegenüberliegend an seinem Außenumfang eine Nut 87 auf. Beide Maßnahmen ermöglichen ein, wenn auch geringfügiges nach innen gerichtetes Spannen des Klemmrings 92, wodurch das Morcellator-Rohr 80 mit Klemmsitz sowohl rotativ wie axial fixiert aufgenommen werden kann. Für diese Verstellmöglichkeit ist jeweils etwa 90^{o} versetzt zum Schlitz 86 eine Bohrung 88 im Rundgriffring 81 vorgesehen, worüber mittels einer Inbus-Schraube 89 und entsprechendem Schraubgewinde ein Vorspannen des Klemmringes 92 nach radial innen möglich ist. Da der Innendurchmesser 90 des Klemmringes bereits Paßsitz mit dem Außenumfang des Morcellator-Rohres 80 besitzt, ist hierdurch sowohl eine sichere, einfache Fixierung, aber auch ein Austausch des Morcellator-Rohres 80 möglich.

## Patentansprüche

1. Zervix-Stanze mit einem hohlzylindrischen Grundkörper (12, 42, 62, 80), der am distalen Ende eine Öffnung (18) mit einem Schneidbereich (20) und am proximalen Ende einen Eingriffsbereich (26, 58, 68, 81) zur Rotation des Grundkörpers relativ zu einer Führungseinrichtung (24, 52, 66) aufweist, wobei der Grundkörper wenigstens in einem proximalen Teil an die Führungseinrichtung (24, 52, 66) angepaßt ist
dadurch **gekennzeichnet,**
daß der Grundkörper (12, 42, 62, 80) ein Rundrohr mit einem über die gesamte Länge gleichbleibenden Außendurchmesser, ist, und daß der Schneidbereich (20) an der distalen Öffnung (18) des als Rundrohr ausgebildeten Grundkörpers (12, 42, 62, 80) einen umlaufenden Wellenschliff aufweist.

2. Zervix-Stanze nach Anspruch 1,
dadurch **gekennzeichnet**,
daß der umlaufende Wellenschliff der distalen Öffnung (18) über den Umfang verteilte Kreisbogenabschnitte mit axial zurücktretenden Wellentälern und axial hervortretenden Wellenbergen aufweist.

3. Zervix-Stanze nach Anspruch 1 oder 2,
dadurch **gekennzeichnet,**
daß der umlaufende Wellenschliff des Schneidbereiches (20) am Außenmantel der distalen Öffnung (18) vorgesehen ist und unterschiedliche Schärfungszonen aufweist.

4. Zervix-Stanze nach einem der Ansprüche 1 bis 3,
dadurch **gekennzeichnet,**
daß das Rundrohr (10, 62, 80) eine hohlzylindrische Führungseinrichtung (12, 22, 66) zur zentrischen Aufnahme eines Führungsstabes (24) aufweist.

5. Zervix-Stanze nach einem der Ansprüche 1 bis 4,
dadurch **gekennzeichnet,**
daß die Führungseinrichtung (12, 22, 66) als Führungszylinder (66) ausgebildet ist, der paßgenau das Rundrohr (80) durchsetzt und insbesondere eine etwas größere Axiallänge als das Rundrohr (80) aufweist.

6. Zervix-Stanze nach einem der Ansprüche 1 bis 5,
dadurch **gekennzeichnet,**
daß der Eingriffsbereich des Rundrohres (80) als Rundgriffring (81) mit einem lösbaren Klemmsitz für das proximale zylindrische Ende des Rundrohres (80) ausgebildet ist.

7. Zervix-Stanze nach einem der Ansprüche 1 bis 6,
dadurch **gekennzeichnet,**
daß zum Drehantrieb des Rundrohres (80) eine Motoreinheit relativ zum Rundrohr (80) fixiert ist.

8. Zervix-Stanze nach einem der Ansprüche 4 bis 7,
dadurch **gekennzeichnet,**
daß ein distaler Teil (14) der Länge des Rundrohres (10) als dünnwandiger Hohlzylinder (14) und ein proximaler Teil (16) der Länge des Rundrohres (10) einen Innendurchmesser oder Bereiche mit einem Innendurchmesser aufweist, der im wesentlichen dem Außendurchmesser des Führungsstabes (24) entspricht.

9. Zervix-Stanze nach Anspruch 8,
dadurch **gekennzeichnet,**
daß sich der dünnwandige Teil (14) des Rundrohres (10) über die distale Hälfte (14) des Rundrohres (10) erstreckt.

10. Zervix-Stanze nach einem der vorhergehenden Ansprüche 4 bis 9,
dadurch **gekennzeichnet,**
daß der Führungsstab (24) eine axial verlaufende Längsnut (30) aufweist, die von der Spitze (28) des Führungsstabes (24) um einen Abstand (d) beabstandet ist.

11. Zervix-Stanze nach einem der Ansprüche 1 bis 10,
dadurch **gekennzeichnet,**
daß das Rundrohr (10) einen distalen Teil (14) und einen proximalen Teil (16) mit unterschiedlichen Innendurchmessern aufweist und
daß der Übergang zwischen den Durchmessern stetig verläuft.

12. Zervix-Stanze nach einem der Ansprüche 1 bis 11,
dadurch **gekennzeichnet,**
daß im Innenbereich der distalen Öffnung (18) des Rundrohres (10) sich proximal erstreckende scharfkantige Klappen ausgebildet sind, die in eine radiale Lage klappbar sind.

13. Zervix-Stanze nach einem der Ansprüche 1 bis 12,
dadurch **gekennzeichnet,**
daß im Rundrohr (10, 40) ein Schraubeinsatz (52) in Form eines Führungszylinders mittels eines Schraubgewindes (50) oder einer schraubenartigen Kulissenführung drehbar und axial beweglich gehalten ist,
daß der Schraubeinsatz (52) eine Führung oder Halterung (54) für den Führungsstab aufweist, und
daß der Schraubeinsatz (52) an dem Führungsstab (24) festlegbar ist.

14. Zervix-Stanze nach Anspruch 13,
dadurch **gekennzeichnet,**
daß der Schraubeinsatz (52) mittels Klemmsitz oder Rastverbindungen an dem Führungsstab (24) festlegbar ist.

15. Zervix-Stanze nach einem der Ansprüche 13 bis 14,
dadurch **gekennzeichnet,**
daß der Schraubeinsatz (52) einen aus dem proximalen Ende des Rundrohres (10, 40) herausragenden Abschnitt mit einem sich radial erstreckenden Griffteil (56) aufweist.

16. Zervix-Stanze nach einem der Ansprüche 5 bis 12,
dadurch **gekennzeichnet,**
daß der Führungszylinder (66) einen aus dem proximalen Ende des Rundrohres (60, 80) herausragenden Abschnitt als Griffteil (70, 82) aufweist.

## Claims

1. Cervical Punch with a hollow cylindrical basic body (12, 42, 62, 80) comprising an opening (18) at its distal end with a cutting reagion (20) and at its proximal end an engagement region (26, 58, 68, 81) for rotating said basic body relatively to guiding means (24, 52, 66), wherein said basic body (12, 42, 62, 80) is adapted to said guiding means (24, 52, 66) at least in a proximal portion,
**characterized** in that
said basic body (12, 42, 62, 80) is a circular tube with an outside diameter remaining constant along its total length and
that said cutting region (20) at said distal opening (18) of said basic body (12, 42, 62, 80) being formed as a circular tube provides an allround wave grinding pattern.

2. Cervical Punch according to claim 1,
**characterized** in that
said allround wave grinding pattern of said distal opening (18) shows segments of a circle distributed over its circumference with axially set back wave valleys and with axially projecting wave crests.

3. Cervical Punch according to claim 1 or 2,
**characterized** in that
that said allround wave grinding pattern of said cutting region (20) is provided at the circumference of the distal opening (18) and shows various zones of sharpness.

4. Cervical Punch according to any of the claims 1 to 3,
**characterized** in that
said circular tube (10, 62, 80) shows hollow cylindrical guiding means (12, 22, 66) for centrically receiving a giude rod (24).

5. Cervical Punch according to any of the claims 1 to 4,
**characterized** in that
said guiding means (12, 22, 66) is formed as a guide cylinder (66) traversing said circular tube (80) in accurately fitting manner and in particular showing a somewhat greater axial length than said circular tube (80).

6. Cervical Punch according to any of the claims 1 to 5,
**characterized** in that
said engagement region of said circular tube (80) is formed as a round gripping ring (81) with a detachable force fit for the proximal cylindrical end of said circular tube (80).

7. Cervical Punch according to any of the claims 1 to 6,
**characterized** in that
for the rotation of said circular tube (80) a motor unit is fixed relatively to said circular tube (80).

8. Cervical Punch according to any of the claims 4 to 7,
**characterized** in that
a distal portion (14) of the length of said circular tube (10) as a thin-walled hollow cylinder (14) and a proximal portion (16) of the length of said circular tube (10) shows an internal diameter or areas with an internal diameter, which substantially corresponds to the external diameter of said guid rod (24).

9. Cervical Punch according to claim 8,
**characterized** in that
said thin-walled portion (14) of said circular tube (10) extends over the distal half of said circular tube (10).

10. Cervical Punch according to any of the claims 4 to 9,
**characterized** in that
said guide rod (24) shows an axially running longitudinal groove (30), which is separated from the tip (28) of said guide rod (24) by a distance (d).

11. Cervical Punch according to any of the claims 1 to 10,
**characterized** in that
said circular tube (10) shows a distal portion (14) and a proximal portion (16) with different internal diameters and in that
the transition between the diameters goes contiously.

12. Cervical Punch according to any of the claims 1 to 11,
**characterized** in that
in the inner region of said distal opening (18) of said circular tube (10) proximally extending sharp-edged laps are formed, which can be swung into a radial position.

13. Cervical Punch according to any of the claims 1 to 12,
**characterized** in that
in said circular tube (10, 40) a screw insert (52) in the form of a guide cylinder is held in a rotably and axially movable manner by means of a screw thread (50) or a srew-like connecting link guide and in that
said screw insert (52) shows a guide or a mounting (54) for said guide rod and in that
said screw insert (52) can be fixed on said guide rod (24).

14. Cervical Punch according to claim 13,
**characterized** in that
said screw insert (52) can be fixed on said guide rod (24) by means of force fitting or locking connections.

15. Cervical Punch according to any of the claims 13 to 14,
**characterized** in that
said screw insert (52) shows a portion projecting out of the proximal end of said circular tube (10, 40) having a radially extending gripping portion (56).

16. Cervical Punch according to any of the claims 5 to 12,
**characterized** in that
said guide cylinder (66) shows a portion projecting out of the proximal end of said circular tube (60, 80) as a grip portion (70, 82).

## Revendications

1. Emporte-pièce avec un corps de base (12, 42, 62, 80) cylindrique creux, qui présente a l'extrémité distale une ouverture (18) avec une zone coupante (20), et à l'extrémité proximale une zone de prise (25, 58, 68, 81) pour la rotation du corps de base par rapport à un dispositif de guidage (24, 52, 66), le corps de base étant ajusté au dispositif de guidage (24, 52, 66) au moins dans une partie proximale, ***caractérisé en ce que*** le corps de base (12, 42, 62, 80) est un tube rond de diamètre extérieur constant sur toute la longueur, et en ce que la zone coupante (20), sur l'ouverture distalc (18) du corps de base (12, 42, 62, 80) configuré en tube rond, présente un polissage périphérique ondulé.

2. Emporte-pièce selon la Revendication 1, ***caractérisé en ce que*** le polissage périphérique ondulé de l'ouverture distale (18) présente des segments d'arc de cercle répartis sur la circonférence, avec des creux d'ondulation en retrait axial et des hauteurs d'ondulation en saillie axiale.

3. Emporte-pièce selon la Revendication 1 ou 2, ***caractérisé en ce que*** le polissage périphérique ondulé de la zone coupante (20) est prévu sur l'enveloppe extérieure de l'ouverture distale (18) et présente différentes zones affûtées.

4. Emporte-pièce selon l'une des Revendications 1 à 3, ***caractérisé en ce que*** le tube rond (10, 62, 80) présente un dispositif de guidage cylindrique creux (12, 22, 66) destiné à recevoir de manière centrée une tige de guidage (24).

5. Emporte-pièce selon l'une des Revendications 1 à 4, ***caractérisé en ce que*** le dispositif de guidage (12, 22, 66) est conformé en cylindre de guidage (66), qui traverse le tube rond (80) en ajustage serré et présente en particulier une longueur axiale un peu plus grande que celle du tube rond (80).

6. Emporte-pièce selon l'une des Revendications 1 à 5, ***caractérisé en ce que*** la zone de prise du tube rond (80) est conformée en bague de saisie ronde (81) avec un ajustement pressé, pouvant être libéré, pour l'extrémité proximale cylindrique du tube rond (80).

7. Emporte-pièce selon l'une des Revendications 1 à 6, ***caractérisé en ce que***, pour l'entraînement en rotation du tube rond (80), une unité de moteur est fixée par rapport au tube rond (80).

8. Emporte-pièce selon l'une des Revendications 4 à 7, ***caractérisé en ce qu***'une partie distale (14) de la longueur du tube rond (10) est conformée en cylindre creux (14) à paroi mince, et une partie proximale (16) de la longueur du tube rond (10) présente un diamètre intérieur, ou des zones d'un diamètre intérieur, qui correspond pour l'essentiel au diamètre extérieur de la tige de guidage (24).

9. Emporte-pièce selon la Revendication 8, ***caractérisé en ce que*** la partie (14) à parois mince du tube rond (10) s'étend sur la moitié distale (14) du tube rond (10).

10. Emporte-pièce selon l'une des Revendications 4 à 9, ***caractérisé en ce que*** la tige de guidage (24) présente une rainure longitudinale (30) s étendant dans la direction axiale, qui est espacée d'une distance (d) de la pointe (28) de la tige de guidage (24).

11. Emporte-pièce selon l'une des Revendications 1 à 10, ***caractérisé en ce que*** le tube rond (10) présente une partie distale (14) et une partie proximale (16) de différents diamètres intérieurs, et en ce que la transition entre les diamètres se fait de manière progressive.

12. Emporte-pièce selon l'une des Revendications 1 à 11, ***caractérisé en ce que*** des volets à bords pointus s'étendant dans le sens proximal sont formés dans la zone à l'intérieur de l'ouverture distale (18) du tube rond (10), lesquels volets sont repliables dans une position radiale.

13. Emporte-pièce selon l'une des Revendications 1 à 12, ***caractérisé en ce qu***'un insert fileté (52) sous la forme d'un cylindre de guidage est maintenu de manière rotative et mobile dans le sens axial dans le tube rond (10, 40) au moyen d'un filetage (50) ou d'un guidage à coulisse de type filetage, en ce que l'insert fileté (52) présente un guidage ou support (54) pour la tige de guidage, et en ce que l'insert fileté (52) peut être immobilisé sur la tige de guidage (24).

14. Emporte-pièce selon la Revendication 13, ***caractérisé en ce que*** l'insert fileté (52) peut être immobilisé au moyen d'un ajustement pressé ou des assemblages à crans sur la tige de guidage (24).

15. Emporte-pièce selon l'une des Revendications 13 à 14, ***caractérisé en ce que*** l'insert fileté (52) présente une section formant saillie depuis l'extrémité proximale du tube rond (10, 40) comprenant une partie de saisie (56) s'étendant dans le sens radial.

16. Emporte-pièce selon l'une des Revendications 5 à 12, ***caractérisé en ce que*** le cylindre de guidage (66) présente une section formant saillie depuis l'extrémité proximale du tube rond (60, 80) en tant que partie de saisie (70, 82).
